Europäisches Patentamt

European Patent Office

Office européen des brevets

(19)

(11) **EP 0 662 154 B1**

(12) **FASCICULE DE BREVET EUROPEEN**

(45) Date de publication et mention
de la délivrance du brevet:
**10.06.1998 Bulletin 1998/24**

(51) Int Cl.$^6$: **C12Q 1/68**, G01N 33/535,
C12Q 1/70

(21) Numéro de dépôt: **93918817.3**

(22) Date de dépôt: **21.09.1993**

(86) Numéro de dépôt international:
**PCT/BE93/00063**

(87) Numéro de publication internationale:
**WO 94/06933 (31.03.1994 Gazette 1994/08)**

(54) **Conjugué destiné à la détection et/ou au dosage d'un composé biologique**

Konjugat für den Nachweis und/oder Quantifizierung von biologischen Komponenten

Conjugate for the detection and/or assay of a biological compound

(84) Etats contractants désignés:
**BE DE ES FR GB IT SE**

(30) Priorité: **22.09.1992 BE 9200827**

(43) Date de publication de la demande:
**12.07.1995 Bulletin 1995/28**

(73) Titulaire: **LA REGION WALLONNE
1040 Bruxelles (BE)**

(72) Inventeurs:
• **REMACLE, José
B-5020 Malonne (BE)**
• **RENTIER, Bernard
B-4613 Taviers (BE)**

• **ALEXANDRE, Isabelle
B-5170 Lesve (BE)**
• **MORRIS, Philippe
B-5000 Namur (BE)**
• **ZAMMATTEO, Nathalie
B-4800 Verviers (BE)**

(74) Mandataire: **Van Malderen, Joelle et al
Office Van Malderen,
Place Reine Fabiola 6/1
1083 Bruxelles (BE)**

(56) Documents cités:
EP-A- 0 123 300         EP-A- 0 137 515
EP-A- 0 254 172         EP-A- 0 304 934
EP-A- 0 362 042         EP-A- 0 431 882

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

LISTE DE SEQUENCES

(1) INFORMATION GENERALE:

(i) DEPOSANT:

(A) NOM: LAMBDATECH S.A.
(B) RUE: LES BEYOLETTES
(C) VILLE: FORRIERES
(E) PAYS: BELGIQUE
(F) CODE POSTAL: 6953
(G) TELEPHONE: 3224263810
(H) TELECOPIE: 3224263760

(ii) TITRE DE L' INVENTION: CONJUGUE DESTINE A LA DETECTION ET/OU AU
    DOSAGE D'UN COMPOSE BIOLOGIQUE

(iii) NOMBRE DE SEQUENCES: 20

(iv) FORME LISIBLE PAR ORDINATEUR:

(A) TYPE DE SUPPORT: Floppy disk
(B) ORDINATEUR: IBM PC compatible
(C) SYSTEME D'EXPLOITATION: PC-DOS/MS-DOS
(D) LOGICIEL: PatentIn Release #1.0, Version #1.25 (OEB)

(2) INFORMATION POUR LA SEQ ID NO: 1:

(i) CARACTERISTIQUES DE LA SEQUENCE:

(A) LONGUEUR: 25 paires de bases
(B) TYPE: acide nucléique
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 1:

TGTGCAACAA TGTGCAGACA TTATA
25

(2) INFORMATION POUR LA SEQ ID NO: 2:

(i) CARACTERISTIQUES DE LA SEQUENCE:

(A) LONGUEUR: 25 paires de bases
(B) TYPE: acide nucléique
(C) NOMBRE DE BRINS: simple
(D) CONFIGURATION: linéaire

(ii) TYPE DE MOLECULE: ADN (génomique)
(xi) DESCRIPTION DE LA SEQUENCE: SEQ ID NO: 2:

ACACGTTGTT ACACGTCTGT AATAT
25

(2) INFORMATION POUR LA SEQ ID NO: 3:

(i) CARACTERISTIQUES DE LA SEQUENCE:

(A) LONGUEUR: 25 paires de bases
(B) TYPE: acide nucléique
(C) NOMBRE DE BRINS: simple
(D) CONFIGURATION: linéaire

(il) TYPE DE MOLECULE: ADN (génomique)

(xi) DESCRIPTION DE LA SEQUENCE: SEQ ID NO: 3:

CCAAGCGTTT AGATGCGTGC CAGGA
25

(2) INFORMATION POUR LA SEQ ID NO: 4:

(i) CARACTERISTIQUES DE LA SEQUENCE:

(A) LONGUEUR: 25 paires de bases
(B) TYPE: acide nucléique
(C) NOMBRE DE BRINS: simple

(D) CONFIGURATION: linéaire

(ii) TYPE DE MOLECULE: ADN (génomique)

(xi) DESCRIPTION DE LA SEQUENCE: SEQ ID NO: 4:

```
                    TACTACATAC ACCCCCGCAC AGACC
                            25
```

(2) INFORMATION POUR LA SEQ ID NO: 5:

(i) CARACTERISTIQUES DE LA SEQUENCE:

(A) LONGUEUR: 25 paires de bases
(B) TYPE: acide nucléique
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 5:

```
                    GGTTCGCAAA TCTACGCACG GTCCT
                            25
```

(2) INFORMATION POUR LA SEQ ID NO: 6:

(i) CARACTERISTIQUES DE LA SEQUENCE:

(A) LONGUEUR: 25 paires de bases
(B) TYPE: acide nucléique
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 6:

```
                    ATGATGTATG TGGGGGCGTG TGTGG
                            25
```

(2) INFORMATION POUR LA SEQ ID NO: 7:

(i) CARACTERISTIQUES DE LA SEQUENCE:

(A) LONGUEUR: 21 paires de bases
(B) TYPE: acide nucléique
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 7:

```
ACCTTAACTG CAGATGTTAT G
                    21
```

(2) INFORMATION POUR LA SEQ ID NO: 8:

(i) CARACTERISTIQUES DE LA SEQUENCE:

(A) LONGUEUR: 21 paires de bases
(B) TYPE: acide nucléique
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 8:

```
TCGAATTGAC GTCTACAATA C
                    21
```

(2) INFORMATION POUR LA SEQ ID NO: 9:

(i) CARACTERISTIQUES DE LA SEQUENCE:

(A) LONGUEUR: 25 paires de bases
(B) TYPE: acide nucléique
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 9:

```
ATATCAGATG ACGAGAACGA AAATG
                      25
```

(2) INFORMATION POUR LA SEQ ID NO: 10:

(i) CARACTERISTIQUES DE LA SEQUENCE:

(A) LONGUEUR: 25 paires de bases
(B) TYPE: acide nucléique
(C) NOMBRE DE BRINS: simple
(D) CONFIGURATION: linéaire

(ii) TYPE DE MOLECULE: ADN (génomique)

(xi) DESCRIPTION DE LA SEQUENCE: SEQ ID NO: 10:

```
TATAGTCTAC TGCTCTTGCT TTTAC
                      25
```

(2) INFORMATION POUR LA SEQ ID NO: 11:

    (i) CARACTERISTIQUES DE LA SEQUENCE:

        (A) LONGUEUR: 25 paires de bases
        (B) TYPE: acide nucléique
        (C) NOMBRE DE BRINS: simple
        (D) CONFIGURATION: linéaire

    (ii) TYPE DE MOLECULE: ARN (génomique)

    (xi) DESCRIPTION DE LA SEQUENCE: SEQ ID NO: 11:

```
                        UGUGCAACAA  UGUGCAGACA  UUAUA
                                   25
```

(2) INFORMATION POUR LA SEQ ID NO: 12:

    (i) CARACTERISTIQUES DE LA SEQUENCE:

        (A) LONGUEUR: 25 paires de bases
        (B) TYPE: acide nucléique
        (C) NOMBRE DE BRINS: simple
        (D) CONFIGURATION: linéaire

    (ii) TYPE DE MOLECULE: ARN (génomique)

    (xi) DESCRIPTION DE LA SEQUENCE: SEQ ID NO: 12:

```
                        ACACGUUGUU  ACACGUCUGU  AAUAU
                                   25
```

(2) INFORMATION POUR LA SEQ ID NO: 13:

    (i) CARACTERISTIQUES DE LA SEQUENCE:

        (A) LONGUEUR: 25 paires de bases
        (B) TYPE: acide nucléique
        (C) NOMBRE DE BRINS: simple
        (D) CONFIGURATION: linéaire

    (ii) TYPE DE MOLECULE: ARN (génomique)

    (xi) DESCRIPTION DE LA SEQUENCE: SEQ ID NO: 13:

```
                        CCAAGCGUUU  AGAUGCGUGC  CAGGA
                                   25
```

(2) INFORMATION POUR LA SEQ ID NO: 14:

    (i) CARACTERISTIQUES DE LA SEQUENCE:

        (A) LONGUEUR: 25 paires de bases
        (B) TYPE: acide nucléique

(C) NOMBRE DE BRINS: simple
(D) CONFIGURATION: linéaire

(ii) TYPE DE MOLECULE: ARN (génomique)

(xi) DESCRIPTION DE LA SEQUENCE: SEQ ID NO: 14:

```
                    UACUACAUAC ACCCCCGCAC AGACC
                               25
```

(2) INFORMATION POUR LA SEQ ID NO: 15:

(i) CARACTERISTIQUES DE LA SEQUENCE:

(A) LONGUEUR: 25 paires de bases
(B) TYPE: acide nucléique
(C) NOMBRE DE BRINS: simple
(D) CONFIGURATION: linéaire

(ii) TYPE DE MOLECULE: ARN (génomique)

(xi) DESCRIPTION DE LA SEQUENCE: SEQ ID NO: 15:

```
                    GGUUCGCAAA UCUACGCACG GUCCU
                               25
```

(2) INFORMATION POUR LA SEQ ID NO: 16:

(i) CARACTERISTIQUES DE LA SEQUENCE:

(A) LONGUEUR: 25 paires de bases
(B) TYPE: acide nucléique
(C) NOMBRE DE BRINS: simple
(D) CONFIGURATION: linéaire

(ii) TYPE DE MOLECULE: ARN (génomique)

(xi) DESCRIPTION DE LA SEQUENCE: SEQ ID NO: 16:

```
                    AUGAUGUAUG UGGGGGCGUG UGUGG
                               25
```

(2) INFORMATION POUR LA SEQ ID NO: 17:

(i) CARACTERISTIQUES DE LA SEQUENCE:

(A) LONGUEUR: 21 paires de bases
(B) TYPE: acide nucléique
(C) NOMBRE DE BRINS: simple
(D) CONFIGURATION: linéaire

(ii) TYPE DE MOLECULE: ARN (génomique)

(xi) DESCRIPTION DE LA SEQUENCE: SEQ ID NO: 17:

ACCUUAACUG CAGAUGUUAU G
21

(2) INFORMATION POUR LA SEQ ID NO: 18:

(i) CARACTERISTIQUES DE LA SEQUENCE:

(A) LONGUEUR: 21 paires de bases
(B) TYPE: acide nucléique
(C) NOMBRE DE BRINS: simple
(D) CONFIGURATION: linéaire

(ii) TYPE DE MOLECULE: ARN (génomique)

(xi) DESCRIPTION DE LA SEQUENCE: SEQ ID NO: 18:

UGGAAUUGAC GUCUACAAUA C
21

(2) INFORMATION POUR LA SEQ ID NO: 19:

(i) CARACTERISTIQUES DE LA SEQUENCE:

(A) LONGUEUR: 25 paires de bases
(B) TYPE: acide nucléique
(C) NOMBRE DE BRINS: simple
(D) CONFIGURATION: linéaire

(ii) TYPE DE MOLECULE: ARN (génomique)

(xi) DESCRIPTION DE LA SEQUENCE: SEQ ID NO: 19:

AUGAUGUAUG UGGGGGCGUG UGUGG
25

(2) INFORMATION POUR LA SEQ ID NO: 20:

(i) CARACTERISTIQUES DE LA SEQUENCE:

(A) LONGUEUR: 25 paires de bases
(B) TYPE: acide nucléique
(C) NOMBRE DE BRINS: simple
(D) CONFIGURATION: linéaire

(ii) TYPE DE MOLECULE: ARN (génomique)

(xi) DESCRIPTION DE LA SEQUENCE: SEQ ID NO: 20:

```
UAUAGUCUAC UGCUCUUGCU UUUAC
                25
```

## Description

### Objet de l'invention

L'invention concerne un conjugué destiné à la détection et/ou au dosage d'un composé biologique. L'invention s'étend également à son procédé d'obtention, à son utilisation dans des procédés de détection et/ou de dosage et à la trousse de diagnostic le comprenant.

### Arrière-plan technologique et état de la technique à la base de l'invention

Beaucoup de procédés de dosages destinés aux tests diagnostiques dans les domaines de la biologie et de la médecine se basent principalement sur la reconnaissance spécifique des anticorps vis-à-vis de leurs antigènes et depuis quelques années sur l'appariement de bases nucléiques (ADN ou ARN). Ces méthodes ont pris des formes diverses en fonction de la complexité des interactions mise en jeu et des moyens d'immobilisation qui sont utilisés pour fixer le ligand à doser (antigène-anticorps ou séquence nucléotidique).

Les techniques de dosage de molécules marquées par un isotope radioactif sont précises et très sensibles. Le désavantage de ces méthodes repose évidemment sur les problème liés à l'installation de pièces spéciales et au stockage des déchets, à la sécurité et à la santé dues à l'installation des isotopes radioactifs, ainsi qu'aux contraintes législatives qui en découlent.

La technique des sondes froides est basée sur l'utilisation de conjugués enzymatiques composés d'une sonde et d'une enzyme liés de manière covalente. Ces conjugués peuvent être détectés par la conversion d'un substrat de l'enzyme en un produit coloré qui est alors mesuré en spectrophotométrie. C'est le cas de la peroxydase ou de la phosphatase alcaline. Bien que ce dosage permette la détection de ligands en quantités importantes, il s'est révélé trop peu sensible dans le cas de dosages immunochimiques - lors de détection d'antigènes présents en faible quantité comme les hormones - et - dans le cas d'hybridations génétiques utilisant des sondes à ADN - où les quantités à détecter sont extrêmement faibles.

La chémoluminescence se base sur l'émission de lumière à partir d'une molécule chimique qui est excitée. Cette excitation peut être réalisée à partir d'une réaction chimique ou par transfert d'énergie d'une autre molécule. Dans le premier cas, la réaction est habituellement catalysée par une enzyme. Les systèmes les plus employés sont soit la peroxydase en présence de luminol, de peroxyde d'hydrogène, soit la phosphatase alcaline en présence de adamantyl 1,2-dioxétane phényl phosphate (L. Kricka, Chim. Chem., 37, 1472-1481, 1991).

Ces systèmes de dosage sont sensibles mais la quantification du signal est très difficile car il est très bref. Une solution serait l'utilisation d'un lanthanide, l'europium, qui une fois excité produit une fluorescence ayant un temps d'excitation particulièrement long et donc détectable. Il a été utilisé pour la détection d'adénovirus avec un seuil de détection de 0,3 pg de DNA (Syvanen et al., Nuc. Acids Res. 14, 1017, 1986).

On peut aussi tirer profit du transfert d'énergie de deux molécules : l'isothiocyanate de fluorescéine (FITC) et l'isothiocyanate de tetraméthyl rhodamine (TRITC). La première (FITC) est fixée sur la sonde et la seconde sur le DNA de l'échantillon. Lorsque la sonde se fixe sur l'échantillon, les deux molécules peuvent se transférer de l'énergie : ainsi l'excitation de la TRITC à une longueur d'onde spécifique va être transférée à la FITC qui se trouve dans son voisinage - si l'appariement a eu lieu- ,et va alors émettre une fluorescence particulière. La méthode est sensible mais de nombreux paramètres doivent encore être optimisés pour une utilisation courante (Davins et al., Analyt. Letters 20, 1897, 1987).

Un nouveau système de dosage en chémoluminescence, commercialisé par la firme Boehringer Mannheim, se base sur la détection de sondes à DNA marquées à la digoxigénine et détectés par des anticorps marqués dirigés contre la digoxigénine (Höltke H. and Kessler C. Nucleic Acids Research, 18 (19), 5843-51, 1990).

Cette molécule est reconnue par un anticorps couplé à la phosphatase alcaline qui réagissant avec un substrat, provoque l'émission d'un photon détectable.

Cette méthode assez sensible présente cependant l'inconvénient de faire intervenir une molécule toxique et d'être difficile à mettre en oeuvre.

La bioluminescence, a pour principe l'émission naturelle de photons provoquée par l'activité enzymatique des luciférases en présence de leurs substrats. Il existe deux types de luciférase, la luciférase utilisant l'ATP comme substrat et la luciférase qui utilise l'oxydation du NADH ou du NADPH comme source d'énergie. Dans ce cas, la luciférase est associée à une NAD(P)H-FMN oxydoréductase.

Ces enzymes permettent la mesure de substrats en quantité très faible ($10^{-12}$ moles). La luciférase à ATP a un rendement quantique 10 fois plus élevé que la luciférase à NAD(P)H, ce qui permet un seuil de détection des substrats 10 fois plus bas. Suivant les conditions de dosage, le signal peut être stable pendant des temps très longs.

Différents procédés utilisant des agents de couplage tels que des billes d'agarose, du N-succinimidyl-3 (2-pyridylthio)proprionate (SPDP), du glutaraldehyde (US.4.604.364) ont été proposés pour lier directement ces enzymes à des ligands tels que des anticorps, des antigènes ou des sondes nucléiques.

La demande de brevet EP 254172 décrit un conjugué anticorps-enzyme (tel qu'une Beta-galactosidase) couplé par un réactif homobifonctionnel (le bis-maléimidopolyalkylène-glycol).

La demande EP 137515 décrit dans un test de bioluminescence, l'utilisation d'un conjugué immoglobuline G - enzyme (aequorin).

Dans ces documents, ces deux conjugués sont obtenus par deux étapes d'activation successives de l'anticorps avec l'agent de couplage et de l'enzyme avec l'agent de couplage.

Cette double activation permet ensuite de faire réagir les deux composants activés entre eux et d'obtenir ainsi un conjugué.

Cependant, ces procédés de diagnostic sont basés sur l'utilisation directe d'une luciférase, qui ne permet pas d'obtenir une sensibilité suffisamment élevée pour la détection et/ou le dosage de certains composés biologiques.

Aussi, d'autres procédés basés sur l'utilisation de kinases, ou de deshydrogénases conjugués à des ligands ont été proposés.

La demande de brevet EP 304934 décrit des conjugués d'enzymes, en particulier de kinases, couplées à des ligands tels que des anticorps, des séquences nucléotidiques, de l'avidine ou de la streptavidine, de manière classique par du gluteraldehyde suivant le procédé décrit par Avrameas et Al. (Bull. of Soc. Chim. Biol. (1968) 50, p. 1169).

Cependant l'utilisation des kinases et des deshydrogénases comme marqueurs a été imitée par leur inactivation due aux agents utilisés pour réaliser le couplage de ces enzymes sur les différents ligands.

La demande de brevet EP 431882 décrit un réactif constitué d'une protéine telle qu'une enzyme (par exemple de la luciférase bactérienne ou une Beta-galactosidase) contenant un groupe thiol préactivé susceptible de fixer par exemple une séquence nucléotidique.

Cependant, ce document ne décrit pas la fixation de ce conjugué préactivé à une kinase ou une deshydrogénase pour être utilisé dans un procédé de détection et/ou de dosage par bioluminescence.

Il est également connu par les demandes de brevets EP 161138 et EP 362042 d'utiliser une glucose 6-phosphate deshydrogénase, fixée de manière covalente à un antigène ou anticorps, à une sonde nucléotidique ou bien à l'avidine pour la détection des sondes biotinylisées. Ce complexe permet de révéler le NADH produit par la glucose 6-phosphate déshydrogénase, la luciférase, ou la FMN-oxydoréductase fixée de manière covalente sur le sépharose® 4B.

Cependant, la sensibilité obtenue par ce procédé est trop faible (sans doute en partie due aux phénomènes d'inactivation lors des couplages des enzymes décrits précédemment), pour détecter de manière optimale certaines séquences nucléotidiques d'organismes pathogènes ou non.

## Buts de l'invention

L'invention consiste à fournir un conjugué et un procédé de préparation de ce conjugué, destiné à la détection et/ou au dosage d'un composé biologique; ledit conjugué étant constitué par un ligand susceptible de se lier de manière spécifique à un composé biologique et par une kinase qui après son couplage avec le ligand conserve son activité enzymatique.

Un autre but de l'invention consiste à fournir un conjugué dont l'enzyme possède une activité spécifique, un "turn over rate" élevé, une stabilité élevée à température ambiante, de faible coût, facilement purifiable et dont la réaction est essentiellement irréversible.

Un but complémentaire de la présente invention consiste à fournir de nouvelles séquences d'acides nucléiques qui ont la propriété de s'apparier à des séquences complémentaires présentes dans le génome des virus responsables des papillomes humains (HPV) et qui puissent être utilisées en tant que ligand d'un conjugué selon l'invention ou être fixées à une molécule qui reconnaît un ligand d'un conjugué selon l'invention.

Un dernier but de la présente invention consiste à fournir une trousse de diagnostic comprenant le conjugué selon l'invention et qui présente une grande sensibilité de détection et/ou de dosage d'un composé biologique; cette sensibilité étant similaire ou plus élevée que pour les trousses de diagnostic de l'état de la technique.

## Eléments caractéristiques de l'invention

L'invention concerne un conjugué actif destiné à la détection et/ou au dosage d'un composé biologique constitué par une kinase, dont les groupements soufres sont de préférence préalablement réduits, et capables de produire un composé intermédiaire utilisable par un système de bioluminescence, de préférence par une luciférase; ladite kinase étant couplée directement à un ligand, préalablement activé par un agent de couplage et susceptible de se lier directement ou indirectement (c'est-à-dire via une cascade de ligands assurant une reconnaissance de type anticorps-anticorps, anticorps - antigène, streptavidine biotine, etc), de manière spécifique audit composé biologique.

Selon l'invention, ladite kinase ou deshydrogénase, du conjugué, n'est pas préalablement activée et est couplée directement à un ligand qui est lui-même préalablement activé par un agent de couplage.

On entend par composé biologique, tout microorganisme pathogène ou non pathogène, tel que les procaryotes, les eucaryotes, les mycoplasmes, les virus, les lignées cellulaires végétales, ou animales, ...; les composants des dits microorganismes ou les composants produits par les dits microorganismes, en particulier les antigènes, les anticorps, les séquences nucléotidiques, éventuellement préalablement amplifiés par une amplification génétique (PCR ou LCR), les lipides, les saccharides, ... et/ou un mélange d'entre eux; de préférence fixés sur un support solide, un gel ou présent en solution.

Avantageusement, la kinase est une pyruvate kinase. De préférence, l'agent de couplage est un agent couplant hétérobifonctionnel tel que le N-succinimidyl 3-2 pyridyldithio proprionate, le succinimidyl 4-(N maléimidométhyl) cyclohéxane-1-carboxylate ou son sulfo-dérivé.

Selon une forme d'exécution préférée de l'invention, le ligand est choisi parmi le groupe constitué par les antigènes, les récepteurs d'hormones, les anticorps, les séquences nucléotidiques, les composés tels que l'avidine ou la streptavidine, capables de se fixer à des séquences nucléotidiques biotinylées et/ou un mélange d'entre eux.

Un autre aspect de la présente invention concerne des conjugués dont le ligand est choisi parmi des séquences nucléotidiques ou des fragments de ces séquences d'ADN monocaténaire ou d'ARN, permettant la détection et/ou le dosage de divers virus responsables de papillomes humains tels que le HPV-6B, le HPV-11, le HPV-16, le HPV-18 et le HPV-33.

Parmi ces séquences sont concernés en particulier :

- tout ou une partie d'une séquence d'ADN de 25 nucléotides:

```
la SEQ ID N°1 : TGTGCAACAA TGTGCAGACA TTATA,
```

- tout ou une partie de sa séquence complémentaire :

```
la SEQ ID N°2 : ACACGTTGTT ACACGTCTGT AATAT,
```

et
- tout ou une partie des séquences d'ARN correspondantes aux séquences susmentionnées:
qui permettent la détection simultanée des 5 types de HPV susmentionnés.

Ces séquences s'hybrident à une région corespondant aux nucléotides 2047-2071 du gène E1 d'HPV6b avec deux mésappariements en position 9 et 15 et à une région correspondant aux nucléotides 2047-2071 du gène E1 d'HPV11 avec deux mésappariements en position 8 et 9, à une région correspondant aux nucléotides 2076-2100 du gène E1 d'HPV16 avec un mésappariement en position 15, à une région correspondant aux nucléotides 2157-2171 du gène E1 d'HPV18 avec un mésappariement en position 6 et à une région correspondant aux nucléotides 2070-2094 du gène E1 d'HPV33 avec trois mésappariements en position 5, 8 et 15.

- tout ou une partie de deux séquences d'ADN de 25 nucléotides :

```
la SEQ ID N°3 : CCAAGGTTT AGATGCGTGC CAGGA
la SEQ ID N°4 : TACTACATAC ACCCCGCAC AGACC
```

- tout ou une partie de leurs séquences complémentaires :

```
la SEQ ID N°5 : GGTTCGCAAA TCTACGCACG GTCCT
la SEQ ID N°6 : ATGATGTATG TGGGGGCGTG TGTGG
```

qui permettent la détection simultanée des HPV de types 6b et 11.

Les séquences 3 et 5 s'hybrident à une région correspondant aux nucléotides 2736-2760 du gène E2 des HPV6b et 11, identiques pour les deux virus.

Les séquence 4 et 6 s'hybrident à une région correspondant aux nucléotides 3355-3379 du gène E2 des HPV6b et 11, identique pour les deux virus.

- tout ou une partie d'une séquence d'ADN des 21 nucléotides:

```
la SEQ ID N°7 : ACCTTAACTG CAGATGTTAT G
```

- tout ou une partie de sa séquence complémentaire :

```
la SEQ ID N°8 : TGGAATTGAC GTCTACAATA C
```

qui permettent la détection simultanée des HPV de types 16, 18 et 33.

Ces séquences s'hybrident à une région correspondant aux nucléotides 6780--6800 du gène L1 d'HPV16 avec un mésappariement en position 15, à une région correspondant aux nucléotides 6759-6779 du gène L1 d'HPV18 avec un mésappariement en position 3 et à une région correspondant aux nucléotides 6734-6754 du gène L1 d'HPV33 avec un mésappariement en position 15.

- tout ou une partie d'une séquence d'ADN de 25 nucléotides:

```
la SEQ ID N°9 : ATATCAGATG ACGAGAACGA AAATG
```

- tout ou une partie de sa séquence complémentaire :

```
la SEQ ID N°10 : TATAGTCTAC TGCTCTTGCT TTTAC
```

qui permettent la détection simultanée des HPV de types 16 et 18.

Ces séquences s'hybrident à une région correspondant aux nucléotides 931-985 du gène E1 d'HPV16 et à une région correspondant aux nucléotides 1007-1031 du gène E1 d'HPV18 avec un mésappariement pour ce virus.

- tout ou une partie des séquences d'ARN correspondant aux séquences susmentionnées :

```
la SEQ ID N°11 : UGUGCAACAA UGUCCAGACA UUAUA
la SEQ ID N°12 : ACACGUUGUU ACACGUCUGU AAUAU
la SEQ ID N°13 : CCAAGCGUUU AGAUGCGUGC CAGGA
la SEQ ID N°14 : UACUACAUAC ACCCCCGCAC AGACC
la SEQ ID N°15 : GGUUCGCAAA UCUACGCACG GUCCU
la SEQ ID N°16 : AUGAUGUAUG UCGGGGCGUG UGUGG
la SEQ ID N°17 : ACCUUAACUG CAGAUGUUAU G
la SEQ ID N°18 : UCGAAUUGAC GUCUACAAUA C
la SEQ ID N°19 : AUAUCAGAUG ACGAGAACGA AAAUG
la SEQ ID N°20 : UAUAGUCUAC UGCUCUUGCU UUUAC
```

Ces séquences ont la propriété de s'apparier à des séquences complémentaires présentes dans le génome de divers types de papillomavirus humains (HPV) et absentes dans le génome humain.

Les hybrides ainsi éventuellement formés peuvent être détectés par diverses méthodes de l'état de la technique telles que hybridation in situ, l'hybridation sur filtre, sur plastiques, en "sandwich", sur support solide, en solution, l'hybridation en Dot blot, Northern blot, Southern blot, par un marquage isotopique ou non isotopique associé au fragment, par la technique des sondes froides, par amplification génétique, notamment par PCR ou LCR et/ou par bioluminescence.

Ces séquences mentionnées permettent l'identification spécifique des HPV les plus fréquemment rencontrés dans le tractus génital (types 6b, 11, 16, 18, 33). Il est connu que certains types de HPV (types 16, 18 et 33) sont plus fréquemment associés à l'apparition d'une tumeur maligne, en l'occurence le cancer du col utérin chez la femme, alors que les autres types (6b et 11) sont caractéristiques de lésions bénignes. La détermination de la présence d'un ou de plusieurs HPV ainsi que du type HPV rencontré, est donc très importante pour l'établissement d'un pronostic et, par conséquent, d'une intervention thérapeutique éventuelle.

De préférence, tout ou une partie des séquences nucléotidiques susmentionnées, éventuellement biotinylées, sont comprises dans le ligand du conjugué selon l'invention ou sont susceptibles de se fixer à un conjugué selon l'invention, pour la détection et/ou le dosage des virus responsables de papillomes humains.

Un autre aspect de l'invention concerne le procédé de préparation du conjugué selon l'invention, dans lequel on fait réagir en solution un agent de couplage, de préférence hétérobifonctionnel, sur un ligand et dans lequel ce ligand activé est couplé à une kinase dont un ou plusieurs ponts sulfure ont été éventuellement réduits.

Avantageusement, la kinase du conjugué selon l'invention est immobilisée au niveau de son site actif sur une colonne d'affinité contenant un gel tel que le Blue sepharose®, ayant une affinité pour la kinase et est couplé au ligand préalablement activé par l'agent de couplage, la colonne est lavée et le conjugué est élué par la colonne en modifiant la composition de la solution de lavage, par exemple en augmentant la force ionique.

Un autre aspect de l'invention concerne également l'utilisation du conjugué selon l'invention pour la détection et/ou le dosage d'un composé biologique; en particulier, l'application pour la détection et/ou le dosage par hybridation de séquences nucléotidiques; de préférence détectées par un procédé choisi parmi le groupe constitué par l'hybridation in situ, l'hybridation sur filtre, sur plastique, sur support solide, en solution, en "sandwich", sur gel, l'hybridation en Dot blot, en Northen blot, Southern blot, par un marquage isotopique ou non-isotopique associé au fragment, par la technique des sondes froides, par amplification génétique, notamment par PCR ou LCR et/ou par un mélange d'entre eux.

Un dernier aspect de l'invention concerne la trousse de détection et/ou de dosage d'un composé biologique comprenant le conjugué selon l'invention et éventuellement les réactifs destinés à la détection et/ou au dosage de séquence nucléotidiques, par un procédé choisi parmi le groupe constitué par l'hybridation in situ, l'hybridation sur filtre, sur plastique, sur support solide, en solution, en "sandwich", sur gel, l'hybridation en Dot blot, en Northen blot, Southern blot, par un marquage isotopique ou non-isotopique associé au fragment, par technique sondes froides, par amplification génétique, notamment par PCR ou LCR et/ou un mélange d'entre eux.

### Brève description des figures

La figure 1 représente de manière schématique l'application du conjugué selon l'invention dans un dosage par hybridation de séquences oligonucléotidiques.

Les figures 2 à 4 représentent des variantes d'exécutions du dosage représenté à la figure 1.

Les figures 5 et 6 représentent respectivement une mesure directe et indirecte du DNA d'un virus HPV par le conjugué selon l'invention.

La figure 7 représente l'effet de la concentration en SPDP sur l'activité de la pyruvate kinase et sur le nombre de bras fixés par molécule d'enzyme.

La figure 8 représente une mesure par bioluminescence avec le conjugué selon l'invention d'IgG humains.

La figure 9 représente une courbe de concentration d'une séquence cible HIV par le conjugué selon l'invention.

### Description d'une forme d'exécution préférée de l'invention

Dans la présente invention, on utilise comme agent révélateur, la luciférase à ATP plutôt que la luciférase à NADH, du fait de sa plus grande sensibilité de dosage des substrats. Afin d'augmenter encore la sensibilité et d'obtenir un signal important sur une longue période de temps, on marque la séquence nucléotidique, l'anticorps, l'antigène, l'avidine ou la streptavidine avec une kinase qui peut produire une quantité constante d'ATP suivant les conditions expérimentales. Cet ATP est ensuite dégradé par la luciférase et l'activité de cette dernière est quantifiée par la détection des photons émis.

### Le choix d'une kinase

L'enzyme marqueur doit être choisi en fonction de différents critères qui sont semblables à ceux utilisés pour les ELISA (Avrameas, Scand. J. Immunol., 8, 7-22, 1978), à savoir:

- une activité spécifique et un "turn over rate" élevés ;

- une stabilité relativement bonne à température ambiante ;
- une perte d'activité, suite au couplage, qui doit être la plus faible possible, et enfin,
- l'enzyme doit être disponible sous forme hautement purifiée,soit commercialement, soit sa préparation doit être relativement simple,
- de plus, l'enzyme doit être choisi en fonction de la stabilité de ses substrats.

D'après Kayne (Kayne F.S., The Enzyme, ed Boyer part. A, 8, 1973, Academic Press, pp. 353-382), la pyruvate kinase est commercialement disponible sous forme très pure mais il est aussi très simple d'en purifier de grandes quantités à partir de muscles de lapin. D'autre part, la réaction catalysée par la kinase est essentiellement irréversible en faveur de la formation de pyruvate et d'ATP car la variation d'énergie libre standard de la réaction est de - 7,5 kcal/mole (Muirhead, M., Biochemical Society Transactions, 15 (5), 996-999, 1987).

$$Phosphoenolpyruvate + ADP \rightarrow Pyruvate + ATP$$

Une solution d'enzyme à 5 mg/ml garde son activité au moins 48 h à température ambiante. La solution commerciale (avec 50% de glycérol) est stable au moins 6 mois à 4°c.

En outre, la pyruvate kinase présente d'autres avantages, tels que sa disponibilité commerciale, sa bonne stabilité thermique et le déplacement de la réaction dans le sens de la formation de l'ATP.

De plus grace à des manipulations génétiques, de nouvelles kinases ayant des propriétés particulières peuvent être produites. Ainsi, on peut produire une pyruvate kinase thermostable grâce au clivage d'une partie de sa séquence. Celle-ci peut alors être utilisée à haute température lors des réactions d'hybridation (Walker et Al. Journal of Molecular Biology (1992) 228, p. 265-276).

De même, d'autres kinases, comme l'acétate kinase peuvent être utilisées.

**Couplage de la pyruvate kinase**

L'utilisation des agents de couplage habituels (glutaraldehyde, SPDP(N-succinimidyl 3-(2-pyridyldithio) propionate), periodate, ...) conduit effectivement à la formation de conjugués pour lesquels la perte d'activité est très élevée et dépasse les 95%. Ceci confirme la sensibilité des kinases aux agents de couplage et le fait que l'utilisation des méthodes de couplage habituelles ne permet pas d'obtenir des conjugués suffisamment actifs pour une utilisation dans les tests diagnostiques.

Lors d'expériences contrôles, les inventeurs ont constaté avec surprise que la kinase native non activée pouvait se fixer de manière covalente sur les ligands activés au SPDP (N-succinimidyl 3-(2-pyridyldithio) propionate). L'explication de cette observation inattendue est probablement que la kinase porte un groupement -SH libre qui peut réaliser un pont disulfure avec le groupement 2 pyridyldisulfure fixé sur la sonde activée. Le conjugué obtenu par cette approche est tout à fait actif.

Les inventeurs ont aussi constaté que l'on peut avantageusement augmenter le rendement du couplage par réduction préalable d'un ou plusieurs ponts disulfures naturels de la kinase par de faibles concentrations en dithiothréitol et cela, sans perte d'activité.

Les conditions de couplage optimalisées en solution sont décrites ci-après pour les anticorps. Les inventeurs ont observé qu'une activation des anticorps avec un excès molaire de 5 SPDP produisait la fixation de 1 à 2 bras SPDP par molécule d'anticorps. Cette concentration en SPDP donne un maximum de conjugués actifs lorsqu'on met en présence une quantité équivalente (en mole) d'anticorps activés et de kinase.

Une autre méthode de couplage sur colonne (phase solide) qui permet d'améliorer le rendement, de faciliter la séparation entre les anticorps conjugués à la kinase et les anticorps non conjugués et de pouvoir préparer une grosse quantité de conjugués, consiste à fixer par des liaisons faibles non covalentes la kinase au niveau de son site actif sur une colonne contenant du Blue Sepharose® (Pharmacia, Uppsala, Suède) puis de faire circuler sur la colonne en circuit fermé et pendant un temps suffisant (habituellement 16h) les anticorps préalablement activés au SPDP.

Les anticorps activés auront ainsi tout le loisir de réaliser des conjugués avec la kinase immobilisée sur le Blue Sepharose®.

Après la réaction, la colonne est lavée et les anticorps qui n'ont pas réagi sont éliminés, puis le conjugué est élué en augmentant la force ionique de la solution de lavage. Cette méthode est décrite en détail ci-après pour les anticorps et pour l'avidine.

La disponibilité de conjugués actifs permet leur utilisation pour des tests diagnostiques basés soit sur des réactions immunologiques telles que les procédés Enzyme linked ImmunoSorbent Assays ou ELISA ou sur l'hybridation de séquences nucléotidiques.

Le principe de la méthode de couplage de la kinase sur les anticorps a pu être étendue à d'autre protéines et même à des oligonucléotides. Ainsi l'avidine et la streptavidine ont pu être activées avec le SPDP puis après élimination du réactif en excès, mis en présence de pyruvate kinase. Le conjugué ainsi formé en solution a été purifié sur une colonne de Blue Sepharose® qui retient la kinase et s'est révélé extrêmement actif.

Pour la fixation de sondes d'oligonucléotides sur la kinase, les sondes à ADN peuvent être terminées par une amine en position 3' et/ou 5' terminal, qui peut être incorporée soit au cours de la synthèse de la chaîne nucléotidique réalisée ou ajoutée par la suite.

Cette amine terminal est alors activée par le SMCC puis après élimination des réactifs en excès mis en présence de pyruvate kinase pour le couplage. Le conjugué est alors facilement purifié par chromatographie moléculaire ou d'affinité.

## Dosages immuno-enzymatiques (ELISA)

La kinase peut être couplée à un anticorps pour former un conjugué anticorps-kinase qui peut servir dans les divers tests ELISA.

Dans le cas d'un dosage direct des conjugués, on utilise une boite multipuits ou des tubes ou un support quelconque sur lesquels sont immobilisés les antigènes.

Les conjugués anticorps-kinase sont alors ajoutés en quantité décroissante. Après lavage, l'activité de la kinase fixée est mesurée en présence de ses substrats, permettant de produire de l'ATP, qui en présence de luciférine est dégradé par la luciférase avec une émission de lumière. Celle-ci peut être mesurée par un bioluminomètre ou par un film sensible comme un Polaroïd® ou encore par un papier négatif sensible aux rayons X. Ce test permet de déterminer le titre des anticorps.

Dans les conditions de dosage de la pyruvate kinase, l'excès de substrats par rapport à la quantité d'enzymes et à la quantité de luciférases est tel que ces enzymes travaillent pendant plusieurs jours. On peut suivre l'émission de lumière pendant trois jours dans un test ELISA normal.

Le test direct permet la mesure de l'antigène grâce à l'utilisation de la méthode dite compétitive. Dans ce cas, l'anticorps est immobilisé sur la boîte ou le support de dosage. Ensuite, l'antigène à doser est incubé en présence d'une quantité fixe d'antigène conjugué à la kinase. Ces deux antigènes vont entrer en compétition pour l'anticorps fixé sur la boîte : plus l'antigène à doser sera en quantité importante, moins l'antigène-kinase pourra se fixer sur l'anticorps. C'est celui-ci qui est détecté par l'activité de la kinase. L'estimation de l'antigène à doser se fait alors en référence à une courbe d'étalonnage.

Le dosage de l'antigène se fait plus facilement grâce à la méthode dite en Sandwich. La boîte ou le support solide contient des anticorps immobilisés. L'ntigène à doser est alors ajouté en dilutions croissantes puis après lavage, une quantité fixe d'un second anticorps marqué à la kinase est ajouté et l'activité de la kinase est mesurée. Une variante à cette méthode consiste à utiliser un second anticorps non marqué et d'ajouter par la suite un anti-anticorps marqué à la kinase (Double Sandwich).

Le dosage en Sandwich permet également la mesure d'anticorps et dans ce cas on utilise un support sur lequel l'antigène est immobilisé. L'anticorps est ajouté en concentrations croissantes puis après lavage, des anti-anticorps marqués à la kinase sont incubés et l'activité de la kinase est mesurée. Ici aussi, on peut réaliser des doubles Sandwich en utilisant un anti-anticorps non marqué puis un deuxième anti-anticorps conjugué à la kinase.

## Dosage par hybridation de séquences oligonucléotidiques

La possibilité d'utiliser l'avidine (ou la streptavidine), les anticorps ou des nucléotides comme sondes qui peuvent être couplés à la kinase, permet la mise au point de tests de reconnaissance de séquences nucléotidiques. Cette technique peut s'appliquer à un très grand nombre de domaines comme les tests diagnostics de détection de bactéries, de champignons, de levures, de virus, de cellules cancéreuses ou de tout organisme biologique possédant une séquence d'acides nucléiques particulière.

Le principe de l'hybridation d'oligonucléotides est bien connu et nous le reprendrons brièvement. Il s'agit, de la reconnaissance d'une séquence de nucléotides (ADN ou ARN) par un brin de nucléotides complémentaire et de leur appariement en une double chaîne dans des conditions de température, pH et sels adéquats. Cette reconnaissance est permise par la formation de ponts hydrogène entre l'adenine (A) et la thymine (T) (ou uracile (U)) et entre la guanine (g) et la cytosine (C). Si l'on connaît la séquence de ADN (ou de RNA) à détecter, il est possible de construire par des procédés chimiques, enzymatiques, ou biologiques la séquence complémentaire qui ira s'hybrider de manière spécifique avec le DNA (ou le RNA) à détecter.

De préférence, cette hybridation s'effectue dans des conditions stringentes, de manière à assurer une hybridation spécifique et éviter ainsi des faux positifs.

L'utilisation des conjugués kinase selon l'invention pour réaliser des tests en bioluminescence a comme avantage une sensibilité très grande et une mesure de détection pouvant s'étendre sur plusieurs jours. Dans le procédé le plus simple, la séquence de nucléotides utilisée pour le test est marquée à la biotine. Ce marquage peut se faire de diverses manières :

- soit chimiquement en utilisant un oligonucléotide biotinylé lors de la synthèse, ou en faisant réagir la séquence avec un NHS-biotine, biotényl aminocapric acid-N-hydroxysuccinimide ester, ou un réactif portant la biotine et susceptible de se fixer sur la séquence nucléotidique,
- soit de façon enzymatique en utilisant la terminal transferase qui incorpore les nucléotides biotinylés à l'extrémité d'une séquence nucléotidique spécifique. La biotine peut être incorporé clans les acides nucléiques grâce à un composé photoactivable appelé photobiotine.

La séquence biotinylée peut alors être reconnue par l'avidine ou la streptavidine elle-même couplée à l'enzyme ou après réaction avec un anticorps marqué à la kinase et dirigé contre la séquence biotinylée, Après hybridation avec la séquence à détecter, un conjugué avidine-kinase est utilisé, qui se fixe sur la biotine. L'activité de la kinase est alors mesurée par sa production d'ATP qui en présence de luciférase émet des photons.

La détection de séquence de l'ADN ou RNA présent en solution est plus complexe car elle nécessite une étape d'immobilisation afin de réaliser un lavage ou une réaction de compétition. Elles peuvent être immobilisées directement sur des filtres de nitrocellulose ou de nylon dans des tests dits de "Dot Blot" ou après séparation par électrophorèse sur gels (Southern blot et Northern blot).

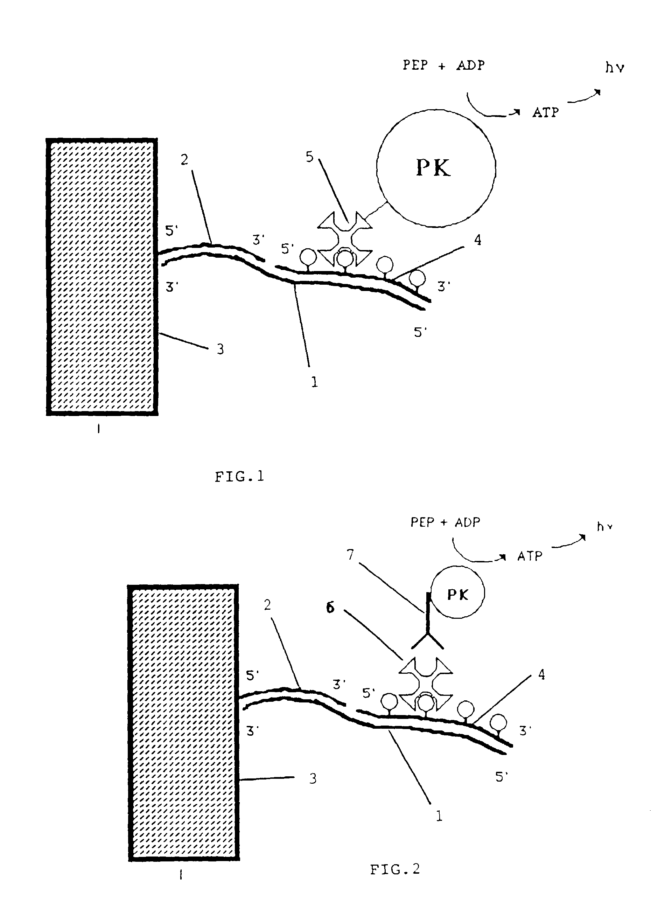

Dans le cas où le DNA 1 (ou RNA) à mesurer se trouve en quantité très faible, on peut aussi immobiliser ce DNA 1 grâce à une séquence complémentaire 2 qui est elle-même immobilisée sur un support 3 (filtre, gel, plastique, verre, billes...) . Le DNA 1 (ou RNA) hybridé sur la sonde immbobilisée, pourra être mesuré à l'aide d'une autre séquence 4 biotinylée et d'un conjugué avidine-kinase 5 ou d'un anticorps kinase anti-sonde. Cette méthode utilisant deux séquences nucléotidiques (2 et 4), - l'une immobilisée dite capteur 2 et l'autre biotinylée dite détecteur 4 - est aussi appelée méthode Sandwich. Du point de vue de la méthodologie, les deux séquences capteur et détecteur peuvent être ajoutées simultanément au DNA (ou RNA) à détecter ou de manière séquentielle suivant les conditions du test et du résultat à obtenir : sensibilité plus grande, rapidité de travail ou facilité des manipulations (figure 1).

Ces méthodes peuvent être complexifiées en utilisant non seulement la reconnaissance entre l'avidine et la biotine mais aussi entre les antigènes (ou haptènes) et les anticorps (figures 2, 3 et 4).

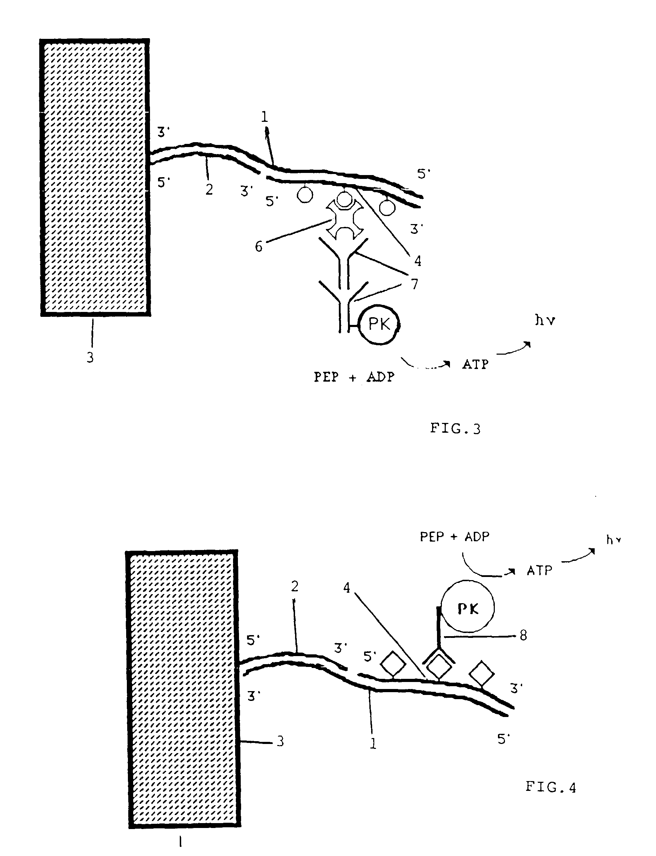

Par exemple, on peut utiliser une séquence d'acide nuclétique biotinylée 4 puis une réaction avec de l'avidine 6, couplée ou non à la kinase, qui sera elle-même reconnue par des anticorps anti-avidine 7 couplés à la kinase (figures 2 et 3).

On peut aussi faire réagir directement (figure 4) des anticorps 8 couplés ou non à la kinase et dirigé contre la biotine qui est fixée sur la sonde. Une autre molécule que la biotine, par exemple la fluoresceine ou la digoxigénine qui peuvent être reconnues par des anticorps spécifiques, peuvent servir à marquer la sonde.

Il existe plusieurs variantes à cette technique comme l'utilisation d'un deuxième anticorps conjugué à la kinase ou l'utilisation d'anticorps conjugué à la biotine sur lesquels on peut faire réagir des conjugués avidine-kinase. Quelle que soit la combinaison utilisée, on utilisera au moins un conjugué avidine-kinase 5 ou anticorps-kinase 7 selon l'invention dans l'une des étapes de la réaction.

Ces reconnaissances spécifiques avidine-biotine ou anticorps-antigène peuvent aussi être mises à profit pour l'immobilisation sur le support solide. Ceci permet de faire la réaction d'hybridation en solution avant de réaliser l'immobilisation. L'avantage de l'hybridation en solution réside en sa plus grande rapidité par rapport à l'hybridation sur support solide pour laquelle les facteurs stériques et de diffusion ralentissent la réaction.

Un procédé particulièrement avantageux consiste à réaliser l'hybridation de l'ADN à mesurer avec deux séquences nucléotidiques complémentaires d'une partie de l'ADN à mesurer. Ces deux séquences sont marquées par des molécules différentes, par exemple l'une par la biotine et l'autre par la kinase. Après hybridation, l'ensemble est mis en présence d'un support portant de l'avidine qui va fixer les séquences biotinylées dont certaines feront partie de l'hybride, qui portera une deuxième séquence-kinase qu'il suffira de mesurer en présence de luciférase et des substrats appropriés.

L'immobilisation peut se réaliser grâce à l'utilisation de séquences primers complémentaires comme des poly C ou poly A qui reconnaissent respectivement des séquences poly C ou poly T (poly U).

La sonde portant la biotine ou une deuxième sonde spécifique DNA ou RNA à doser, peut porter une séquence poly A (ou poly T ou poly G) qui pendant l'hybridation en solution se fixera sur la séquence complémentaire fixée à un support.

Les deux séquences peuvent aussi porter une biotine ou un antigène (ou haptène) comme du dinitrophénol ou de la fluorescéine.

Après hybridation, on peut immobiliser l'hybride sur un support portant l'avidine et révéler l'hybride par un anticorps dirigé contre l'antigène ou l'haptène (ici, anti-dinitrophénol ou anti-digoxigenine) marqué à la kinase. Si l'anticorps n'est pas marqué à la kinase, un deuxième anti-anticorps marqué à la kinase peut être utilisé.

L'immobilisation peut aussi se faire à partir d'anticorps fixés sur le support qui va reconnaître et fixer l'antigène ou l'haptène (ici le dinitrophénol). La révélation de l'hybride se fera alors en faisant réagir de l'avidine-kinase sur la séquence biotinylée de l'hybride. Les autres méthodes de mise en évidence de la biotine-avidine expliquées ci-dessus peuvent aussi servir mais dans tous les cas, un conjugué-kinase au moins sera utilisé.

On peut aussi coupler la kinase à des anticorps reconnaissant les doubles brins de DNA.

Dans ces situations de détection des hybrides décrites ci-dessus, on faisait appel, dans une des étapes au moins, à la reconnaissance entre la biotine et l'avidine ou des anticorps et leur antigènes ou haptènes.

On peut éviter le passage par cette réaction dans le cas notamment de la pyruvate kinase (on a observé que cette enzyme était stable à 45°c pendant 2h au moins, ce qui représente des conditions d'hybridation que l'on peut obtenir notamment avec des oligonucléotides). Sur base de ces observations, on réalise un conjugué composé de kinase directement couplée sur la séquence d'oligonucléotides qui servira à l'hybridation; l'activité de la kinase étant mesurée en présence de ses substrats et de luciférase.

Cette technique peut être appliquée après amplification d'une séquence par PCR en solution ou même dans une cellule fixée. Dans ce cas, le procédé utilisant une pyruvate kinase thermostable est particulièrement bien adapté.

## EXEMPLES

## TESTS IMMUNOLOGIOUES

I- Mise en évidence de conjugués anti-IgG-kinase.

Des IgG de souris sont immobilisés sur des plaques multipuits. Les puits sont alors saturés par l'albumine bovine pendant 4 h. Ensuite, les puits sont lavés 3 fois avec une solution de Tween®. Les puits peuvent éventuellement être séchés. Les anticorps conjugués sont alors incubés en présence d'albumine pendant 2 h à 20°C. Après lavage : l'activité de la kinase est dosée en ajoutant une solution mélange contenant ses substrats à savoir : du phosphénolpyruvate, de l'ADP, la luciférine, la luciférase ces deux derniers étant destinées à la détection de l'ATP produit à partir de l'ADP et du phosphoénolpyruvate par la kinase. La mesure des photons se fait dans un bioluminomètre au cours des 5 minutes qui suivent, et le résultat est exprimé en fonction de plateau ou de l'intégration pendant 1 minute. On peut aussi exposer à l'aide d'un film polaroïd sensible, par exemple 3.000 ou 20.000 ASA pendant 5 minutes suivi d'un développement de 45 sec. Dans un test réalisé de cette manière, les anti-IgG-kinase ont été dilués successivement et ont pu être détectés jusqu'à une dilution de 2.560 fois, ce qui représentait $2,6. \cdot 10^{-13}$ moles d'anticorps par test.

II- Mesure d'IgG considérés comme antigènes (méthode Sandwich).

La même expérience fut réalisée comme au point précédent mais en immobilisant sur les plaques multipuits des anticorps de lapins anti-IgG humains. Ces plaques ont été incubées avec des IgG humaines à des dilutions de plus en plus grandes, ensuite ont été lavées, puis des anticorps de souris anti-IgG-humaine conjugués à la kinase ont été ajoutés à tous les puits. La mesure de la kinase par la plaque photographique a permis de détecter une concentration de $8,3.10^{-14}$ mole/test d'IgG humaine.

III- Détermination du titre des anticorps anti-Beta-HCG (human chorionic gonadotropin).

On réalise deux dilutions de 20X et de 100X d'un sérum positif anti-Beta-HCG contenant 98.000 mUI/ml avec du sérum négatif (provenant d'un sujet humain de sexe féminin). Ces deux dilutions dont été incubées pendant 60 minutes à 37°C et sous atmosphère humide dans 2 X 3 séries de micropuits préalablement coatés avec l'anticorps monoclonal trappeur (anti-Beta HCG). Après trois rinçages, on incube une préparation commerciale d'IgG polyclonaux de lapin anti-HCG, dialysée et diluée 100 X, 1.000 X et 10.000X. On rince trois fois dans le tampon phosphate. Enfin, pour chaque série de micropuits, nous avons réalisé une courbe de concentration en conjugués qui sont composés d'un anticorps de mouton anti-IgG de lapin conjugués à la pyruvate kinase. Après trois rinçages, on dose l'activité au moyen du lecteur de plaques en bioluminescence. Si on dilue l'anticorps de lapin anti-HCG 100X et si on utilise une dilution de 50 fois pour le conjugué, on observe une activité de 30 RLU pour la dilution 20X du serum HCG à 98.000 mUI/ml, et de 7 RLU (valeur du test moins la valeur du blanc) pour la dilution 100X. On peut donc doser des concentrations de 4.900 mUI/ml de BetaHCG dans le serum avec un rapport signal/bruit de 7 et une concentration de 980 mUI/l avec un rapport signal bruit de 2. Cette valeur constituerait, dans l'état actuel des choses, la limite de détection du dosage. Un sérum est considéré comme positif lorsqu'il contient plus de 2.000 mUI/l ce qui cadre bien avec les exigences requises en laboratoire de biologie clinique. Une optimalisation des conditions permettrait d'améliorer encore les performances de ce dosage.

IV- Dosage des anticorps anti-histone.

Un dosage d'anticorps anti-histone dans le sérum d'un patient positif a été réalisé en diluant le sérum de 2 en 2 fois par un sérum contrôle pour les dilutions allant de 800 à 102.400 fois. Les boîtes multipuits ont été "coatées" avec des protéines histones. Le sérum humain anti-histone a été incubé dans ces puits, puis après lavage un anticorps de lapin anti-IgG humain marqué à la kinase. Ce sérum anti-histone donnait un signal linéaire en fonction de la dilution jusqu'à une dilution de 25.600 fois alors que le même test réalisé avec un conjugué marqué à la peroxydase et révélé en spectrophotométrie suivant les conditions habituelles du fabricant (Biolal, Belgique) en utilisant l'ABTS (azino-benzthiazoline sulfonate) comme substrat ne détectait que jusqu'à la dilution de 1.600 fois.

## Tests génétiques

I. Mesure directe du DNA du virus HPV-18

A. Fixation covalente du DNA sur les boîtes plastiques

Le DNA du virus d'HPV-18 a été immobilisé de manière covalente sur des boîtes de plastique multipuits modifiées de manière à ce qu'elles présentent des groupements aminés en surface. La fixation se fait entre le phosphate 5' terminal de l'ADN et la fonction amine greffée sur le plastique, via les agents couplants que sont l'1-éthyl-3-(3-diméthylaminopropyl)-carbodiimide et le méthylimidazole.

Les plaques sont incurvées 5 h à 50°C, puis les puits sont lavés par une solution 5 x SSC contenant 0,25% de SDS et à 50°c. Ces conditions de fixation sont recommandées par la firme qui commercialise ces boîtes Covalink®. (NUNC, Gibco BRL).

B. Révélation du DNA par des oligo-biotinylés

Un oligonucléotide de 21 paires de base a été sélectionné pour sa spécificité à reconnaître une séquence spécifique des DNA viraux de HPV 18 - 33 et 16. Il s'agit de la séquence :

$$5'- A\ C\ T\ T\ T\ A\ A\ C\ T\ G\ C\ A\ G\ A\ T\ G\ T\ T\ A\ T\ G\ -3'$$

Cette séquence s'hybride à une région correspondant aux nucléotides 6780-6800 du gène L1 HPV16 avec un mésappariement en position 15 et un en position 3, à une région correspondant aux nucléotides 6759-6779 du gène L1 d'HPV18 et à une région correspondant aux nucléotides 6734-6754 du gène L1 d'HPV 33 avec un mésappariement en position 15 et un mésappariement en position 3.

Le marquage à la biotine de cet oligonucléotide se fait au moyen de la terminal transferase. Celle-ci fixe à l'extremité 3' quelques d.UTP biotinylés et quelques d-CTP (non biotinylés).

L'hybridation se déroule dans les puits où l'ADN à été fixé, dans une solution d'hybridation comprenant l'oligo biotinylé et l'on incube 16h à 50°C.

Après lavage, le conjugué avidine-kinase est alors ajouté à raison de 0,01 mg/ml et après lavage, l'activité de la kinase est révélée en présence de ses substrats et de la luciférase comme explicitée dans les exemples ci-dessus. La quantité de DNA viral de HPV-18 mise en évidence dans ces conditions correspond à la solution de 100 ng/puits, ce qui est très élevé.

II- Mesure indirecte du DNA clu virus HPV-18 (Méthode Sandwich) par deux oligonucléotides

A. Génome de 586 BP

Pour éviter les problèmes de viscosités, d'interactions, d'encombrements qu'impliquent la manipulation du génome complet d'HPV (+/- 8 000 bp), on amplifie par PCR une partie de ce génome, à savoir, un brin de 586 bp à partir de la 6193e base jusqu'à la 6779e base.

B. Fixation covalente de l'oligo-trappeur

L'oligonucléotide "trappeur" de 21 paires de bases a une spécificité unique pour le génome d'HPV 18 et constitue le complémentaire des 21 premières bases à partir de l'extrémité 3' du fragment sélectionné ci-dessus. La séquence est

5'-T T T T C.C A A G A T G G T G A T A T G G- 3'

L'oligo-trappeur est immobilisé de manière covalente dans les boîtes NUNC® de la même manière que précédemment pour le DNA du virus HPV 18.

On incube durant 5h à 50°C, 5 ng d'oligonucléotides par puits et fixe environ 1,2 ng par un lien covalent.

C. Hybridation du fragment d'HPV 18

Pour l'hybridation, les puits contenant l'oligonucléotide trappeur sont d'abord rincés.

On ajoute alors à 200 microl/puits de la solution d'hybridation contenant clos concentrations décroissantes du fragment du génome d'HPV 18 diluée dans l'eau et dénaturé. L'hybridation se déroule à 50°c pendant 2 h.

D. Hybridation de l'oligonucléotides biotinylé

L'oligonucléotide détecteur de 21 paires de bases est spécifique pour les génomes d'HPV 16, 18 et 33 (cfr. mesure indirecte du virus HPV 18 - point B)et constitue le complémentaire des 21 dernières bases à partir de l'extrémité 3' du fragment de 586 paires de bases sélectionné au pointA. Sa séquence:

5'-A C T T T A A C T G C A G A T G T T A T G-3'

Après avoir lavé les puits, nous procédons, à une nouvelle hybridation de la même manière que précédemment sinon que l'on ajoute à la solution d'hybridation les oligonucléotides biotinylés. Cette hybridation dure 1 h à 50°C.

E. Révélation par bioluminescence

Après hybridation, les plaques sont lavées, puis le conjugué avidine-kinase est ajouté à raison à 0,01 mg/ml et après lavage, l'activité de la kinase est révélée en présence de ses substrats et de la luciférase, comme explicitée dans les exemples ci-dessus.

III. Mesure indirecte du DNA de virus HPV (méthode sandwich en présence de chaîne d'ADN par double reconnaissance de sondes plus longues et détection par l'intermédiaire d'anticorps marqués à la kinase)

A. Génome de 586bp

Pour éviter les problèmes de viscosité d'interactions d'encombrement qu'implique la manipulation de génome complet d'HPV, on amplifie par PCR une partie du génome, à savoir un brin de 586bp à partir de la 6193ème base jusqu'à la 6779ème base.

B. Fixation covalente d'une sonde trappeur

Une sonde "trappeur" fabriquée par PCR et phosphorylée en 5' est fixée dans les puits de façon covalente comme préalablement.

C Hybridation sandwich

Une sonde de détection biotinylée à été produite par PCR. La biotine est incorporée grâce à la présence de dUTP biotinylé en rapport 1/2 avec le dTTP.

L'hybridation se fait dans les puits où la sonde trappeur est immobilisée. La solution d'hybridation comprend l'ADN cible de HPV de 586bp et la sonde biotinylée par PCR. L'hybridation se fait à 70°C pendant 2 heures.

D. Révélation

Après lavage et saturation, l'avidine est incubée dans les puits, puis l'anticorps anti-avidine suivi du l'anticorps dirigé contre ce dernier et marqué à la kinase. Après lavage et équilibration, l'activité de la kinase est révélée en présence de ses substrats et du système luciférase comme explicité ci-dessus et on mesure la production de lumière au luminoskan ou on prend une photo Polaroïd®.

E. Résultats

La mesure de la lumière était de 10000 RLU (Relative light unit) sur le bioluminomètre Lumac® pour 500pg de DNA cible de HPV et de 2500 RLU pour 50pg de DNA cible de HPV.

IV. Mesure indirecte du DNA de virus HPV (méthode sandwich en présence de chaîne d'ADN et révélation par un conjugué streptavidine kinase)

La préparation des sondes trappeurs fixées dans les puits en plastique et des sondes détectrices marquées à la biotine est identique que dans l'exemple III ci-dessus. L'hybridation est réalisée dans les mêmes conditions. Après hybridation, on lave et on sature les puits avant d'incuber le conjugué streptavidine kinase dilué 1000X. Les puits sont lavés 4 fois avant d'être incubés en présence des substrats de la kinase et la luciférase comme dans les exemples précédents. Dans ces conditions, une courbe de concentration a été réalisée de 500pg à 5pg en DNA cible de HPV et les valeurs de RLU sont supérieures aux contrôles réalisés en présence de DNA non spécifique.

V. Mesure indirecte du DNA de virus HIV (méthode sandwich en présence de chaîne d'ADN et révélation par un conjugué streptavidine kinase).

A. Cible produit par PCR.

Un segment de la chaîne de RNA du virus du sida (HIV-I) a été transcript en DNA puis amplifié par PCR. Ce fragment comprend 473bases situé entre la 1214ème base et la 1687ème.

B. Hybridation

La préparation des sondes trappeurs et détectrices ainsi que l'hybridation et la révélation se font de la même façon que dans le cas VIII.

C. Résultats

Une courbe de concentration en fragments de DNA cible de HIV a été réalisée allant de 1000 à 1pg. Pour chacune de ces concentrations la mesure de RLU était significativement supérieure aux contrôles réalisés avec du DNA non spécifique (résultats présentés à la figure 9).

VI. Mesure indirecte de DNA de virus cytomégalovirus

Un segment de la chaîne de RNA du virus de CMV a été transcript en DNA puis amplifié par PCR. Ce fragment de 434bases est situé entre la 2223ème base et la 2657ème base. Le protocole expérimental fut réalisé comme pour l'exemple VIII et on a obtenu une courbe de concentration en DNA cible de CMV allant de 1000 à 10pg.

VII. Mesure indirecte de DNA de la bactérie Chlamydia trachomatis

Un segment de DNA de chlamydia trachomatis a été amplifié par PCR. Ce fragment de 415 bases est situé après la 5339ème base et constitue le DNA cible.
Le protocole expérimental fut réalisé comme pour l'exemple VIII et on a obtenu une courbe de concentration en DNA cible de Chlamydia trachomatis allant de 2000 à 20pg.

VIII. Elisa en bioluminescence avec lecture sur plaque Polaroïd®

De nouveaux conjugués sont synthétisés, des plaques 96 puits sont coatées avec des IgG de lapin anti-IgG humains, des IgG humains et des IgG de souris.

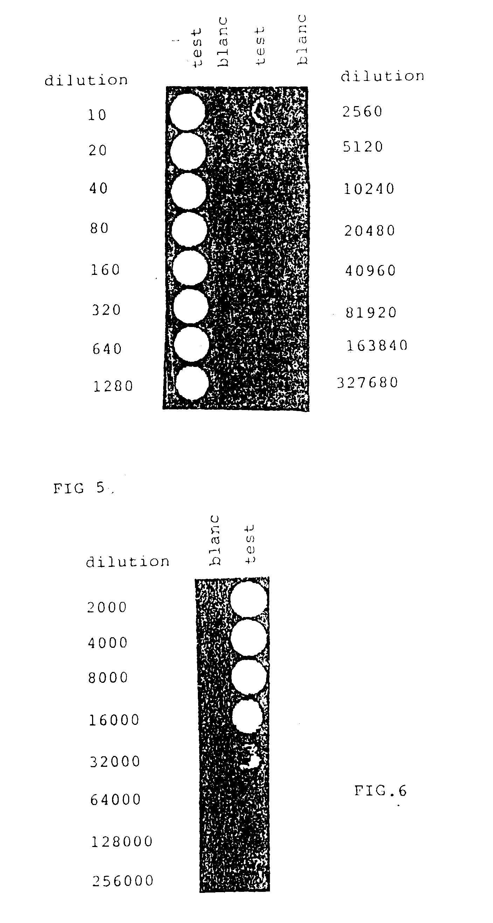

a/La première opération consiste à réaliser une courbe de saturation afin de déterminer le titre des conjugués;
b/Le dosage Elisa proprement dit a été réalisé suivant deux méthodes qui varient légèrement au niveau du dosage de l'enzyme marqueur. Dans un premier cas, mesure directe, on mesure continuellement l'ATP produit par la réaction de la pyruvate kinase en bioluminescence. Cette méthode permet de détecter $2,6.10^{-13}$ mole/test d'IgG humains (fig 5). Dans un deuxième cas, mesure indirecte, l'ATP produit est accumulé pendant 60 minutes puis mesuré en bioluminescence. Dans ce cas, on détecte $8,32.10^{-14}$ mole/test (fig.6). On constate que la sensibilité n'a pas été améliorée comme on aurait pu l'espérer.

IX. Effet de la concentration en SPDP sur le nombre de bras fixés et sur l'activité de la pyruvate kinase, protection par les substrats

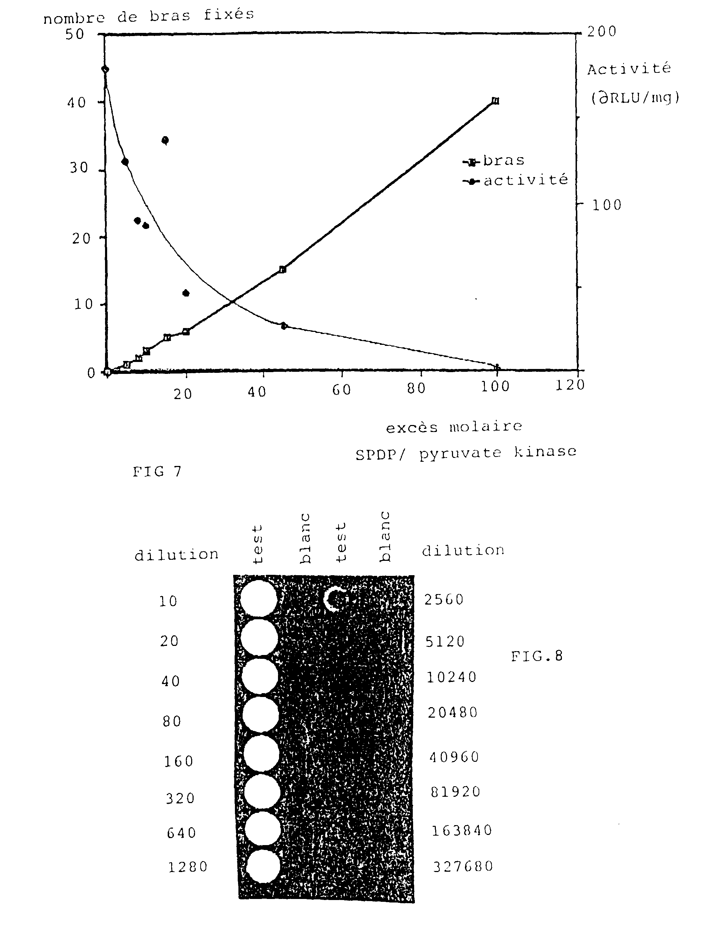

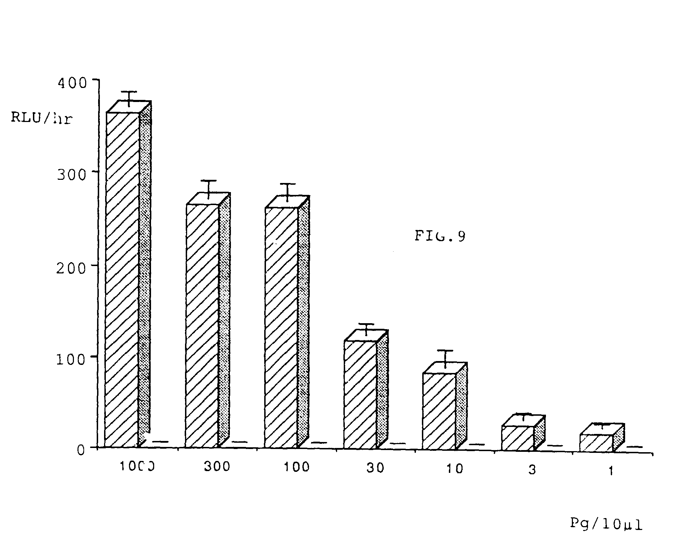

Des aliquots de 100ml de pK à 10mg/ml dans duKH2PO4 100mM pH7 sont activés avec 200 ml de SPDP en concentration croissante dans de l'éthanol. Après 30 minutes de réaction, le SPDP en excès est éliminé sur Sephadex® G25 et la PK est analysée par rapport à son activité résiduelle et au nombre de bras fixés par molécule d'enzyme. L'expérience est aussi réalisée en présence de substrats en concentration 1mM (ADP phosphoénolpyruvate et MgCl2). Le graphique montre que pour un faible nombre de bras fixés sur l'enzyme (inférieur à 5), l'activité résiduelle est semblable si l'expérience est effectuée en présence de substrats ou non. Au delà d'un excès de 10 SPDP, ce qui correspond à +/- 4 bras fixés par enzyme, l'activité résiduelle est légèrement supérieure à l'expérience en présence des substrats (fig.7). Dans une boîte micropuits, des immunoglobines (IgG) de souris (blanc) et humains (test) sont "coatées" selon une méthode classique. Après une incubation avec des conjugués IgG anti-IgG humains couplés à la pyruvate kinase, les micropuits sont lavés et on révèle en luminescence l'activité de la kinase couplée à l'IgG anti-IgG humain. Ces derniers se fixent dans les puits contenant leur antigène, à savoir les IgG humains. (fig.8).

X. Mesure de fragment de DNA produits à partir du RNA de virus HIV

Dans une boite Covalink (NUNC) de micropuits, des sondes à DNA spécifiques du virus HIV ont été fixées de manière covalente par leur extrémité 5' puis incubées en présence de fragments de DNA provenant d'une amplification par PCR (Polymerase Chain Reaction) d'un fragment 475bp correspondant à une partie du RNA du virus du SIDA (HIV-I). Les solutions contiennent également une sonde détectrice de 205bp biotinylée et aussi complémentaire de la séquence d'ADN du virus HIV-I.

Après hybridation à 70°C et lavage, un conjugué streptavidine-kinase est incubé avec les hybrides et après lavage, les substrats de la kinase et la luciférase sont ajoutés afin de mesurer les conjugués kinase-strepdavidine présent dans les puits. La figure 9 montre la pente de l'augmentation de lumière en RLU (Relative Light Unit) observé par heure d'incubation de ces réactifs. Les contrôles ont été réalisés avec un ADN cible de 571bp provenant du génome de souris.

Les tests ont été faits en 3 exemplaires et représentent la moyenne. Les écarts types (± s) sont indiqués sur la figure. La figure montre que 1 pg de DNA peut être détecté significativement par ce test.

**Revendications**

1.  Conjugué actif destiné à la détection et/ou au dosage d'un composé biologique, caractérisé en ce qu'il est constitué par une kinase dont les groupes soufres sont de préférence préalablement réduits, et capable de produire un composé intermédiaire utilisable par un système de bioluminescence, de préférence par une luciférase, ladite kinase étant couplée à un ligand, préalablement activé par un agent de couplage, et susceptible de se lier directement ou indirectement, de manière spécifique au dit composé biologique.

2.  Conjugué selon la revendication 1 caractérisé en ce que la kinase porte un groupement -SH libre et est couplée par ce groupement au ligand préalablement activé par l'agent de couplage.

3.  Conjugué selon la revendication 1 ou 2 caractérisé en ce que la kinase est une pyruvate kinase.

4.  Conjugué selon les revendications 1 à 3 caractérisé en ce que l'agent de couplage est hétérobifonctionnel.

5.  Conjugué selon la revendication 4 caractérisé en ce que l'agent de couplage est le N-succinimidyl 3- (2-pyridyldithio) proprionate, le succinimidyl 4- (N-maleimidométhyl) cyclohexane -1 - carboxylate ou son sulfodérivé.

6.  Conjugué selon l'une quelconque des revendications précédentes caractérisé en ce que le ligand est choisi parmi les groupes constitués par les antigènes, les récepteurs d'hormones, les anticorps, les séquences nucléotidiques, les composés capables de se fixer à des séquences nucléotidiques biotinylées et/ou un mélange d'entre eux.

7.  Conjugué selon la revendication 6 caracterisé en ce que le ligand est de l'avidine ou de la streptavidine, éventuellement modifiée.

8.  Conjugué selon l'une des revendications 6 ou 7, caractérisé en que la séquence nucléotidique comprend tout ou une partie d'une séquence nucléotidique choisie parmi le groupe constitué par les séquences nucléotidiques suivantes:

```
la SEQ ID N°1 : TGTGCAACAA TGTGCAGACA TTATA.
la SEQ ID N°2 : ACACGTTGTT ACACGTCTGT AATAT
la SEQ ID N°3 : CCAAGCGTTT AGATGCGTGC CAGGA
```

```
la SEQ ID N°4 : TACTACATAC ACCCCCGCAC AGACC
la SEQ ID N°5 : GGTTCGCAAA TCTACGCACG GTCCT
la SEQ ID N°6 : ATGATGTATG TGGGGGCGTG TGTGG
la SEQ ID N°7 : ACCTTAACTG CAGATGTTAT G
la SEQ ID N°8 : TGGAATTGAC GTCTACAATA C
la SEQ ID N°9 : ATATCAGATG ACGAGAACGA AAATG
la SEQ ID N°10 : TATAGTCTAC TGCTCTTGCT TTTAC
la SEQ ID N°11 : UGUGCAACAA UGUGCAGACA UUAUA
la SEQ ID N°12 : ACACGUUGUU ACACGUCUGU AAUAU
la SEQ ID N°13 : CCAAGCGUUU AGAUGCGUGC CAGGA
la SEQ ID N°14 : UACUACAUAC ACCCCCGCAC AGACC
la SEQ ID N°15 : GGUUCGCAAA UCUACGCACG GUCCU
la SEQ ID N°16 : AUGAUGUAUG UGGGGGCGUG UGUGG
la SEQ ID N°17 : ACCUUAACUG CAGAUGUUAU G
la SEQ ID N°18 : UGGAAUUGAC GUCUACAAUA C
la SEQ ID N°19 : AUAUCAGAUG ACGAGAACGA AAAUG
la SEQ ID N°20 : UAUAGUCUAC UGCUCUUGCU UUUAC
```

**9.** Procédé de préparation du conjugué selon l'une quelconque des revendications précédentes caractérisé en ce que on fait réagir en solution un agent de couplage, de préférence hétérobifonctionnel sur un ligand et en ce que ce ligand activé est couplé à une kinase de préférence par un groupement -SH libre de la kinase dont un ou plusieurs ponts sulfures ont été éventuellement réduits.

**10.** Procédé de préparation du conjugué selon la revendication 9 caractérisé en ce que la kinase ou la déshydrogénase du conjugué est immobilisé au niveau de son site actif sur une colonne contenant un gel, de préférence le Blue Sépharose®, ayant une affinité pour la kinase ou la déshydrogénase et est couplée au ligand préalablement activé par un agent de couplage et en ce que la colonne est lavée et le conjugué est élué de la colonne en changeant la composition de la solution de lavage, de préférence en augmentant la force ionique.

**11.** Application du conjugué selon l'une quelconque des revendications 1 à 8 pour la détection et/ou le dosage d'un composé biologique.

**12.** Application selon la revendication 11 pour la détection et/ou le dosage par hybridation de séquences nucléotidiques.

**13.** Application selon la revendication 12 pour la détection et/ou le dosage de séquences nucléotidiques, préalablement détectées par un procédé choisi parmi le groupe constitué par l'hybridation in situ, l'hybridation sur filtre, sur plastique, sur support solide, en solution, en "sandwich" sur gel, l'hybridation en Dot blot, Northern blot, Southern blot, par un marquage isotopique ou non-isotopique associé au fragment, par la technique des sondes froides, par amplification génétique, notamment par PCR ou LCR et/ou par un mélange d'entre eux.

**14.** Trousse de détection et/ou de dosage d'un composant biologique caractérisé en ce qu'il comprend le conjugué selon l'une quelconque des revendications 1 à 8.

**15.** Trousse de diagnostic selon la revendication 14 caractérise en ce qu'elle comprend également les réactifs destinés à la détection et/ou au dosage de séquences nucléotidiques par un procédé choisi parmi le groupe constitué par l'hybridation in situ, l'hybridation sur filtre, sur support solide, en solution, en "sandwich" sur gel, l'hybridation en Dot blot, Northen blot, Southern blot, par un marquage isotopique ou non-isotopique associé au fragment, par la technique des sondes froides, par amplification génétique, notamment par PCR ou LCR et/ou un mélange d'entre eux.

**Patentansprüche**

1. Aktive Verbindung mit konjugierten Doppelbindungen, die zum Nachweis und/oder zur quantitativen Bestimmung einer biologischen Verbindung bestimmt ist, dadurch gekennzeichnet, daß sie aus einer Kinase besteht, deren Schwefelgruppen vorzugsweise vorher reduziert werden, und die fähig ist, eine Zwischenverbindung zu bilden, die von einem Biolumineszenzsystem, vorzugsweise einer Luciferase, verwendet werden kann, wobei die Kinase an einen Liganden gekoppelt ist, der vorher durch ein Kopplungsmittel aktiviert wurde, und fähig ist, sich direkt oder indirekt auf spezifische Weise an die biologische Verbindung zu binden.

2. Verbindung mit konjugierten Doppelbindungen, gemäß Anspruch 1, dadurch gekennzeichnet, daß die Kinase eine freie SH-Gruppe aufweist, und über diese Gruppe mit dem vorher durch das Kopplungsmittel aktivierten Liganden gekoppelt ist.

3. Verbindung mit konjugierten Doppelbindungen, gemäß Anspruch 1 oder 2, dadurch gekennzeichnet, daß die Kinase eine Pyruvatkinase ist.

4. Verbindung mit konjugierten Doppelbindungen, gemäß Anspruch 1 bis 3, dadurch gekennzeichnet, daß das Kopplungsmittel heterobifunktionell ist.

5. Verbindung mit konjugierten Doppelbindungen, gemäß Anspruch 4, dadurch gekennzeichnet, daß das Kopplungsmittel N-Succinimidyl-3-(2-pyridyldithio)-propionat, Succinimidyl-4-(N-maleimidomethyl)-cyclohexan-1-carboxylat oder das Sulfoderivat davon ist.

6. Verbindung mit konjugierten Doppelbindungen, gemäß irgendeinem der vorhergehenden Ansprüche, dadurch gekennzeichnet, daß der Ligand aus den Gruppen gewählt wird, die gebildet wird von Antigenen, Hormonrezeptoren, Antikörpern, Nukleotidsequenzen, Verbindungen, die fähig sind, sich an biotinylierte Nukleotidsequenzen zu binden, und/oder einem Gemisch davon.

7. Verbindung mit konjugierten Doppelbindungen, gemäß Anspruch 6, dadurch gekennzeichnet, daß der Ligand eventuell modifiziertes Avidin oder Streptavidin ist.

8. Verbindung mit konjugierten Doppelbindungen, gemäß irgendeinem der Ansprüche 6 oder 7, dadurch gekennzeichnet, daß die Nukleotidsequenz ganz oder teilweise eine Nukleotidsequenz aufweist, die aus der Gruppe gewählt ist, die von den folgenden Nulkeotidsequenzen gebildet wird:

```
der SEQ ID Nr. 1 : TGTGCAACAA TGTGCAGACA TTATA

der SEQ ID Nr. 2 : ACACGTTGTT ACACGTCTGT AATAT

der SEQ ID Nr. 3 : CCAAGCGTTT AGATGCGTGC CAGGA

der SEQ ID Nr. 4 : TACTACATAC ACCCCCGCAC AGACC
```

```
der SEQ ID Nr.  5 :  GGTTCGCAAA TCTACGCACG GTCCT

der SEQ ID Nr.  6 :  ATGATGTATG TGGGGGCGTG TGTGG

der SEQ ID Nr.  7 :  ACCTTAACTG CAGATGTTAT G

der SEQ ID Nr.  8 :  TGGAATTGAC GTCTACAATA C

der SEQ ID Nr.  9 :  ATATCAGATG ACGAGAACGA AAATG

der SEQ ID Nr. 10 :  TATAGTCTAC TGCTCTTGCT TTTAC

der SEQ ID Nr. 11 :  UGUGCAACAA UGUGCAGACA UUAUA

der SEQ ID Nr. 12 :  ACACGUUGUU ACACGUCUGU AAUAU

der SEQ ID Nr. 13 :  CCAAGCGUUU AGAUGCGUGC CAGGA

der SEQ ID Nr. 14 :  UACUACAUAC ACCCCCGCAC AGACC

der SEQ ID Nr. 15 :  GGUUCGCAAA UCUACGCACG GUCCU

der SEQ ID Nr. 16 :  AUGAUGUAUG UGGGGGCGUG UGUGG

der SEQ ID Nr. 17 :  ACCUUAACUG CAGAUGUUAU G

der SEQ ID Nr. 18 :  UGGAAUUGAC GUCUACAAUA C

der SEQ ID Nr. 19 :  AUAUCAGAUG ACGAGAACGA AAAUG

der SEQ ID Nr. 20 :  UAUAGUCUAC UGCUCUUGCU UUUAC
```

9. Verfahren zur Herstellung der Verbindung mit konjugierten Doppelbindungen gemäß irgendeinem der vorhergehenden Ansprüche, dadurch gekennzeichnet, daß man ein vorzugsweise heterobifunktionelles Kopplungsmittel in Lösung auf einen Liganden einwirken läßt, und daß dieser aktivierte Ligand vorzugsweise durch eine freie SH-Gruppe der Kinase mit einer Kinase, bei der eventuell eine oder mehrere der Schwefelgruppen reduziert wurden, gekoppelt wird.

10. Verfahren zur Herstellung der Verbindung mit konjugierten Doppelbindungen, gemäß Anspruch 9, dadurch gekennzeichnet, daß die Kinase oder die Dehydrase der Verbindung mit konjugierten Doppelbindungen im Bereich ihres aktiven Zentrums auf einer Säule, die ein Gel, vorzugsweise Blue Sepharose®, enthält, das eine Affinität für die Kinase oder die Dehydrase hat, immobilisiert wird, und mit dem vorher durch ein Kopplungsmittel aktivierten Liganden gekoppelt wird, und daß die Säule gewaschen wird und die Verbindung mit konjugierten Doppelbindungen aus der Säule eluiert wird, indem die Zusammensetzung der Waschlösung geändert wird, vorzugsweise, indem die Ionenstärke erhöht wird.

11. Anwendung der Verbindung mit konjugierten Doppelbindungen gemäß irgendeinem der Ansprüche 1 bis 8 zum Nachweis und/oder zur quantitativen Bestimmung einer biologischen Verbindung.

12. Anwendung gemäß Anspruch 11 zum Nachweis und/oder zur quantitativen Bestimmung durch Hybridisierung von Nukleotidsequenzen.

13. Anwendung gemäß Anspruch 12 zum Nachweis und/oder zur quantitativen Bestimmung von Nukleotidsequenzen, die vorher durch ein Verfahren nachgewiesen wurden, das aus der Gruppe gewählt wurde, die gebildet wird von der Hybridisierung in situ, der Hybridisierung auf Filter, auf Kunststoff, auf festem Träger, in Lösung, mit "Sandwich" auf Gel, der Hybridisierung mit Dot-Blot, Northern-Blot, Southern-Blot, von einer mit dem Fragment kombinierten Isotopenmarkierung oder Nicht-Isotopen-Markierung, von der Technik der kalten Sonden, von der genetischen Amplifizierung, insbesondere durch PCR oder LCR, und/oder von einem Gemisch.

14. Ausrüstung zum Nachweis und/oder zur quantitativen Bestimmung eines biologischen Bestandteils, dadurch gekennzeichnet, daß er die Verbindung mit konjugierten Doppelbindungen gemäß irgendeinem der Ansprüche 1 bis 8 aufweist.

15. Diagnoseausrüstung gemäß Anspruch 14, dadurch gekennzeichnet, daß sie ebenfalls die Reagenzien aufweist, die bestimmt sind für den Nachweis und/oder die quantitative Bestimmung von Nukleotidsequenzen nach einem Verfahren, das aus der Gruppe gewählt wird, die gebildet wird von der Hybridisierung in situ, der Hybridisierung auf Filter, auf Kunststoff, auf festem Träger, in Lösung, mit "Sandwich" auf Gel, der Hybridisierung mit Dot-Blot, Northern-Blot, Southern-Blot, von einer mit dem Fragment kombinierten Isotopenmarkierung oder Nicht-Isotopen-Markierung, von der Technik der kalten Sonden, von der genetischen Amplifizierung, insbesondere durch PCR oder LCR, und/oder von einem Gemisch davon.

## Claims

1. Active conjugate intended for detecting and/or assaying a biological compound, characterised in that it comprises a kinase whose sulphur groups are preferably previously reduced and which is capable of producing an intermediate compound, which can be used by a bioluminescence system, preferably by a luciferase, the said kinase being coupled to a ligand which is previously activated by a coupling agent and which is able specifically to bind, directly or indirectly, to the said biological compound.

2. Conjugate according to the claim 1, characterised in that the kinase carries a free -SH group and is coupled by said group to the ligand being previously activated by the coupling agent.

3. Conjugate according to the claim 1 or 2, characterised in that the kinase is a pyruvate kinase.

4. Conjugate according to any one of the preceding claims, characterised in that the coupling agent is heterobifunctional.

5. Conjugate according to the claim 4, characterised in that the coupling agent is N-succinimidyl-3-(2-pyridyldithio) propionate, succinimidyl-4-(N-maleimido-methyl) cyclohexane-1-carboxylate or its sulpho derivative.

6. Conjugate according to any one of the preceding claims, characterised in that the ligand is selected from among the group comprising antigens, hormone receptors, antibodies, nucleotide sequences and compounds capable of binding to biotinylated nucleotide sequences, and/or a mixture thereof.

7. Conjugate according to the claim 6, characterised in that the ligand is avidin or streptavidin, possibly being modified.

8. Conjugate according to the claim 6 or 7, characterised in that the nucleotide sequence comprises all or part of a nucleotide sequence selected from among the group comprising the following nucleotide sequences :

```
the SEQ ID NO.  1 : TGTGCAACAA TGTGCAGACA TTATA

the SEQ ID NO.  2 : ACACGTTGTT ACACGTCTGT AATAT

the SEQ ID NO.  3 : CCAAGCGTTT AGATGCGTGC CAGGA

the SEQ ID NO.  4 : TACTACATAC ACCCCCGCAC AGACC

the SEQ ID NO.  5 : CGTTCGCAAA TCTACGCACG GTCCT

the SEQ ID NO.  6 : ATGATGTATG TGGGGGCGTG TGTGG

the SEQ ID NO.  7 : ACCTTAACTG CAGATGTTAT G

the SEQ ID NO.  8 : TGGAATTGAC GTCTACAATA C

the SEQ ID NO.  9 : ATATCAGATG ACGAGAACGA AAATG

the SEQ ID NO. 10 : TATAGTCTAC TGCTCTTGCT TTTAC

the SEQ ID NO. 11 : UGUGCAACAA UGUGCAGACA UUAUA

the SEQ ID NO. 12 : ACAGCUUGUU ACACGUCUGU AAUAU

the SEQ ID NO. 13 : CCAAGCGUUU AGAUGCGUGC CAGGA

the SEQ ID NO. 14 : UACUACAUAC ACCCCCGCAC AGACC

the SEQ ID NO. 15 : GGUUCGCAAA UCUACGCACG GUCCU

the SEQ ID NO. 16 : AUGAUGUGUG UGGGGGCGUG UGUGG

the SEQ ID NO. 17 : ACCUUAACYG CAGAUGUUAU G

the SEQ ID NO. 18 : UGGAAUUGAC GUCUACAAUA C

the SEQ ID NO. 19 : AUAUCAGAUG ACGAGAACGA AAAUG

the SEQ ID NO. 20 : UAUAGUCUAC UGCUCUUGCU UUUAC
```

**9.** Process for preparing the conjugate according to any of the preceding claims, characterised in that a coupling agent, which is preferably heterobifunctional, is reacted, in solution, with a ligand, and in that this activated ligand is coupled to a kinase, preferably by the free -SH group of the kinase, one ore more sulphide bridges of which have, where appropriate, been reduced.

**10.** Process of preparing the conjugate according to the claim 9, characterised in that the kinase or the dehydrogenase of the conjugate is immobilised at its active site on a column containing a gel, preferably a Blue Sepharose ®, possessing an affinity for the kinase, and is coupled to the previously activated ligand by a coupling agent, and in that the column is washed and the conjugate is eluted from the column by changing the composition of the washing solution, preferably by increasing the ionic strength.

**11.** Use of the conjugate according to any of the claims 1 to 8, for detecting and/or assaying a biological compound.

**12.** Use according to the claim 11, for detecting and/or assaying nucleotide sequences by means of hybridisation.

**13.** Use according to the claim 12, for detecting and/or assaying nucleotide sequences which are previously detected by a method selected from among the group comprising in situ hybridisation, hybridisation on a filter, on plastic, on a solid support, in solution, in a "sandwich" or on a gel, dot blot, Northern blot or Southern blot hybridisation, the use of an isotopic or non-isotopic label attached to the fragment, the use of cold probes technique or the use of genetic amplification, especially using PCR or LCR, and/or the use of a mixture thereof.

14. Kit for detecting and/or assaying a biological compound, characterised in that it includes the conjugate according to any of the claims 1 to 8.

15. Diagnostic kit according to the claim 14, characterised in that it also includes the reagents intended for detecting and/or assaying nucleotide sequences by a method selected from among the group comprising in situ hybridisation, hybridisation on a filter, on plastic, on a solid support, in solution, in a "sandwich" or on a gel, dot blot, Northern blot or Southern blot hybridisation, the use of an isotopic or non-isotopic label attached to the fragment, the use of cold probes technique or the use of genetic amplification, especially using PCR or LCR, and/or the use of a mixture thereof.

FIG.1

FIG.2

FIG.3

FIG.4

test blanc test blanc

| dilution | | dilution |
|---|---|---|
| 10 | | 2560 |
| 20 | | 5120 |
| 40 | | 10240 |
| 80 | | 20480 |
| 160 | | 40960 |
| 320 | | 81920 |
| 640 | | 163840 |
| 1280 | | 327680 |

FIG 5.

blanc test

| dilution | |
|---|---|
| 2000 | |
| 4000 | |
| 8000 | |
| 16000 | |
| 32000 | |
| 64000 | |
| 128000 | |
| 256000 | |

FIG.6

nombre de bras fixés

FIG 7

FIG.8

FIG.9